# EUROPEAN PATENT APPLICATION

(11) **EP 4 082 433 A1**
(43) Date of publication of application: **02.11.2022**
(21) Application number: 20906463.3
(22) Date of filing: 23.12.2020
(51) Int. Cl.: A61B 5/08, G10L 25/51

(54) **CONTROL DEVICE, CONTROL SYSTEM, AND CONTROL METHOD**

(30) Priority: 27.12.2019 JP 2019238533
(71) Applicant: Kyocera Corporation, Kyoto-shi Kyoto 612-8501 (JP)
(72) Inventor: IKEDA, Yutaka, Kyoto-shi, Kyoto 612-8501 (JP)
(74) Representative: TBK
(86) International application number: PCT/JP2020/048140
(87) International publication number: WO 2021/132320

(57) **Abstract**

To enable accurately determining, based on a sound emitted by an inspection target, a classification of the sound. A control apparatus (1) according to an embodiment includes a classification information acquiring unit (13) that acquires classification information of a sound, a sound acquiring unit (11) that acquires a sound data including information of the sound, a storage unit (20) that stores definition data (25), an extraction unit (12) that extracts a plurality of features of the sound data, and a model construction unit (15) that constructs a learned model where machine learning, based on the plurality of features of the sound data and the classification information, on a correlation between the plurality of features and the classification of the sound is performed.

## Description

### Technical Field

The present invention relates to a control apparatus, a control system, and a control method.

### Background Art

Conventionally, there is a technique for identifying, based on a sound emitted by a biological body or an object, the characteristics of the sound. The biological body is, for example, a human or an animal. For example, Patent Literature 1 discloses a technique for digitally representing an auscultatory sound to map a relationship between the auscultatory sound and a disease.

### Citation List

### Patent Literature

Patent Literature 1: JP 2007-508899 T

### Summary of Invention

### Technical Problem

As described above, there are a variety of techniques for finding characteristics of a sound emitted by a biological body or an object to classify the sound, based on some perspective. In such a technique, more accurate determination on the classification of the sound is demanded.

### Solution to Problem

A control apparatus according to an embodiment includes a first data acquiring unit that acquires first data including information indicating a sound classification of a sound, a second data acquiring unit that acquires second data including sound information of the sound, a storage unit that stores definition data, for extracting a plurality of features from the second data, of the plurality of features, an extraction unit that extracts the plurality of features of the second data based on the definition data, and a model construction unit that constructs a learned model where machine learning, based on the plurality of features of the second data and the first data, on a correlation between the plurality of features and the sound classification is performed.

A control apparatus according to an embodiment includes a second data acquiring unit that acquires second data including information of a sound of an inspection target, a storage unit that stores definition data, for extracting a plurality of features from the second data, of the plurality of features, an extraction unit that extracts the plurality of features of the second data based on the definition data, and an estimation unit that estimates a classification of the sound of the inspection target from the plurality of features of the second data by using a learned model where machine learning on a correlation between the plurality of features and the classification of the sound is performed.

A control system according to an embodiment includes a control apparatus and a detection apparatus that transmits the second data detected to the control apparatus.

A control method according to an embodiment includes the steps of acquiring first data including information indicating a sound classification of a sound, acquiring second data including sound information of the sound, storing definition data, for extracting a plurality of features from the second data, of the plurality of features, extracting the plurality of features of the second data based on the definition data, and constructing a learned model where machine learning, based on the plurality of features of the second data and the first data, on a correlation between the plurality of features and the sound classification is performed.

A control method according to an embodiment includes the steps of acquiring second data including information of a sound of an inspection target, storing definition data, for extracting a plurality of features from the second data, of the plurality of features, extracting the plurality of features of the second data based on the definition data, and estimating a classification of the sound of the inspection target from the plurality of features of the second data by using a learned model where machine learning on a correlation between the plurality of features and the classification of the sound is performed.

### Advantageous Effects of Invention

According to an aspect of the invention according to the present disclosure, it is possible to accurately determine, based on a sound emitted by the inspection target, the classification of the sound.

### Brief Description of Drawings

FIG. 1 is a block diagram illustrating a configuration of a main part of a control system according to a first embodiment.
FIG. 2 is a diagram illustrating an extraction process of a feature from a time waveform of sound data.
FIG. 3 is a diagram illustrating an extraction process of a feature from a frequency waveform of sound data.
FIG. 4 is a diagram illustrating an extraction process of a feature from a spectrogram waveform of sound data.
FIG. 5 is a diagram illustrating an example of a data structure of a feature list.
FIG. 6 is a schematic view illustrating an overview of input and output data of a learned model and a configuration of the learned model.
FIG. 7 is a flowchart illustrating a flow of model constructing processing.
FIG. 8 is a block diagram illustrating a configuration of a main part of a control system according to a second embodiment.
FIG. 9 is a block diagram illustrating a configuration of a main part of a control system according to a third embodiment.
FIG. 10 is a flowchart illustrating a flow of estimation processing.
FIG. 11 is a block diagram illustrating a configuration of a main part of a control system according to a fourth embodiment.

### Description of Embodiments

The related art for classifying sounds have classified the sounds by simply applying known classifications to the sounds. In such a related art, the sound is not always clearly classified. For example, assume that a breath sound is classified from a medical perspective. In this case, noises included in the breath sound may include a noise having a plurality of characteristics mixed and/or a noise that does not match the known classification but is actually caused by a disease. In the related art, the classification of these noises has been difficult to accurately determine.

The inventors found problems in the related art like this. The present disclosure describes a control apparatus, a control system, a control method, and the like that can accurately determine the classification of the sound.

A control system according to an embodiment is a system for constructing a learned model capable of estimating, from digital data (sound data) of a sound emitted by a biological body such as a human or an object, a classification of the sound. In addition, the control system according to an embodiment can use the learned model to estimate, from sound data of a sound emitted from a person or object as an inspection target, the classification of the sound.

An application range of the invention according to the present disclosure is not particularly limited. For example, the control system according to an embodiment may construct a learned model capable of estimating a classification of a breath sound of a human or animal. Additionally, the control system according to an embodiment may use the learned model to estimate a classification of a breath sound of a human or animal as an inspection target.

The "classification of sound" may be, for example, a medical classification of a sound obtained when a specialist such as a physician auscultates a human or an animal. The sound may be, for example, a sound generated by a flow of gas in a human or animal body. That is, the sound may be classified into a sound generated, for example, with a breathing motion. In particular, the sound may be classified into a lung sound, for example. The sound may be classified into, for example, a breath sound and an adventitious sound among sounds included in the lung sounds.

The breath sound may be further classified into, for example, a normal breath sound and an abnormal sound. The normal sound may be further classified into, for example, a vesicular breath sound, a bronchoalveolar breath sound, a bronchial breath sound, and a tracheal breath sound. The abnormal sound may be further classified into characteristics, for example, decrease, absence, prolonged expiration, and a bronchial-like breath sound. Alternatively, the abnormal sound may be further classified into, for example, a stenotic sound. The stenotic sound may be further classified for each stenosed site such as a trachea, a pharynx, and larynx, for example.

The adventitious sound may be further classified into, for example, a rale (i.e., rhonchus) and other sounds. The rale may be further classified into, for example, a continuous rale and a discontinuous rale. The continuous rale may further be classified into, for example, a sonorous rhonchus, a wheeze, a squawk, and a stridor. The discontinuous rale may be further classified into, for example, a coarse discontinuous rale, a fine discontinuous rale, a bubbling rale, and a crepitant rale. Other sounds may be further classified into, for example, a pleural friction rub and a pulmonary vascular noise.

Note that the classification of sound is not limited to these examples. That is, the classification of sound may include any classification used medically concerning a sound. In addition, in the present disclosure, sounds classified into the sound generated with a breathing motion, such as a breath sound and an adventitious sound, may be collectively referred to simply as breath sounds.

For example, the control system according to an embodiment may construct a learned model capable of estimating the classification of a sound obtained when a building material or the like is struck. The control system may use the learned model to estimate a classification of a sound obtained when a building material as an inspection target is struck. In this case, the "classification of sound" may include, for example, a type of classification for a case that a durability of the building material is specified from the sound, which has been identified based on the experience of a specialist (such as an architect), for example.

Hereinafter, various aspects of the present invention will be described based on the first to fourth embodiments. Note that in the following embodiments, a case that the sound is a human breath sound, and the classification of sound is a medical classification of a breath sound will be described.

### First Embodiment

### System Overview

A control system 100 according to an embodiment is a system for constructing a learned model where machine learning on a correlation between a plurality of "features of sound" and a "classification of sound" based on data (first data) including information indicating a classification of a sound and data (second data) of the sound is performed. The first data may include information indicating a classification of a breath sound, for example. The second data may include, for example, data obtained by electronically converting the breath sound by a conventionally known technique using a conventionally known sound collector such as an auscultator, a microphone, and the like. That is, the second data may be data in a digital format including sound information on the sound. That is, the control system 100 according to an embodiment may be, for example, a system for constructing a learned model capable of estimating a medical classification of a breath sound. The control system 100 can calculate, from data of sound (sound data) obtained by electronically converting a sound that can be collected using a conventionally known sound collector such as an auscultator, a microphone, and the like by a conventionally known technique, a plurality of features of the collected sound. The control system 100 may acquire a result of medical classification by a specialist (e.g., a physician, a nurse, and the like) for a recorded sound. The control system 100 according to an embodiment can calculate a plurality of features from data of a breath sound, for example. The control system 100 may also acquire a result of medical classification of the breath sound. Note that a sound is not limited to a breath sound. A sound may be, for example, a heart sound associated with heart beats and a borborygmus sound associated with a movement of a stomach or bowel. That is, a sound is, for example, a sound caused by some physiological or pathological phenomenon.

The control system 100 can create training data that includes a plurality of calculated features as input data and medical classification results as correct answer data. The control system 100 may perform machine learning on a learning model using the training data. By doing so, the control system 100 can construct a learned model where learning on the correlation between the feature of sound and the classification of sound is performed.

### Configuration of Main Part

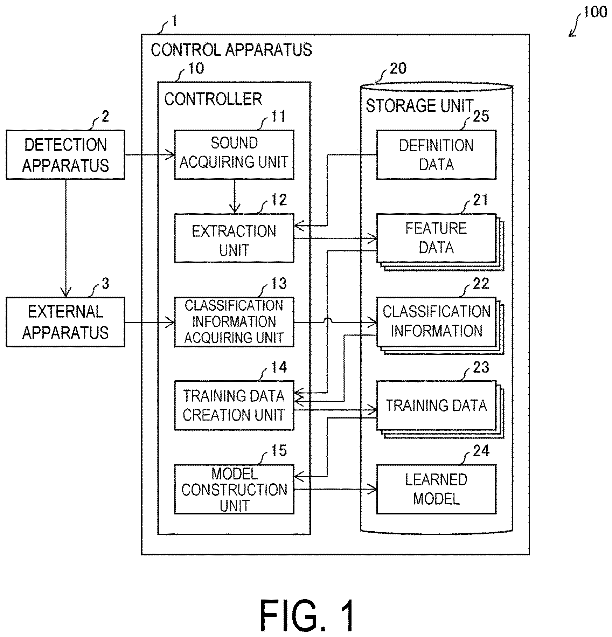

FIG. 1 is a block diagram illustrating a configuration of a main part of the control system 100. The control system 100 includes a control apparatus 1, a detection apparatus 2, and an external apparatus 3. The control apparatus 1 and the detection apparatus 2, the control apparatus 1 and the external apparatus 3, and the detection apparatus 2 and the external apparatus 3 may each be connected by wire or wirelessly.

### Detection Apparatus 2

The detection apparatus 2 is an apparatus for detecting a breath sound. For example, the detection apparatus 2 can be implemented by a directional microphone. In the present embodiment, the detection apparatus 2 can detect the second data. The detection apparatus 2 can transmit the detected sound data to a sound acquiring unit 11 in the control apparatus 1. The detection apparatus 2 may transmit the sound data to the external apparatus 3. Note that the detection apparatus 2 may be implemented by an auscultator or the like having a built-in microphone. The detection apparatus 2 may have a function of recording the detected breath sound. In this case, the detection apparatus 2 may include a storage medium such as memory.

### External Apparatus 3

The external apparatus 3 is an input apparatus for a specialist such as a physician to input the first data. A specific configuration of the external apparatus 3 is not particularly limited. For example, the external apparatus 3 may be implemented by a personal computer (PC), a smartphone, or the like. The external apparatus 3 includes an input unit for a specialist to input information indicating a classification of a breath sound and a communication unit for the external apparatus 3 to communicate with another apparatus. The external apparatus 3 may include a sound output unit outputting the sound data and an interface for connecting an external recording medium such as a flash memory or an SD card.

When the external apparatus 3 receives the sound data from the detection apparatus 2, the external apparatus 3 may output the sound data from the sound output unit. The specialist operating the external apparatus 3 may listen to the sound data to determine a classification of the breath sound and input a determination result to the external apparatus 3 via the input unit. The external apparatus 3 may transmit the acquired determination result to the control apparatus 1. The "determination result" referred to here can be said to be the first data including information indicating a classification of a breath sound corresponding to the sound data. At this time, the external apparatus 3 may transmit, to the control apparatus 1, the information indicating the classification of the breath sound to which identification information of the sound data of the breath sound is added. A format of the identification information is not particularly limited. For example, a file name of the sound data may be the identification information, or a creation time of the sound data may be the identification information. Hereinafter, information indicating the classification of the sound (i.e., the breath sound in the present embodiment) to which the identification information of the sound data is added is referred to as "classification information".

Note that in a case that the detection apparatus 2 has a function to listen to a breath sound, the specialist may listen to the breath sound using the detection apparatus 2 to input the result (i.e., the determination result of the classification of the breath sound) to the external apparatus 3. In this case, the external apparatus 3 need not output the sound data. The external apparatus 3 may read and output the sound data from the external recording medium connected to the external apparatus 3. The external apparatus 3 may record the classification information on the external recording medium connected to the external apparatus 3.

### Control Apparatus 1

The control apparatus 1 is an apparatus for constructing a learned model 24 based on the sound data and the classification of the breath sound corresponding to the sound data. The control apparatus 1 includes a controller 10 and a storage unit 20. Note that the control apparatus 1 may include an interface capable of connecting an external recording medium. The control apparatus 1 may include an input unit such as a button, a mouse, and a touch panel, and a display unit such as a display.

The controller 10 comprehensively controls the control apparatus 1. The controller 10 includes the sound acquiring unit (second data acquiring unit) 11, an extraction unit 12, a classification information acquiring unit (first data acquiring unit) 13, and a training data creation unit 14.

The sound acquiring unit 11 acquires sound data from the detection apparatus 2. The sound acquiring unit 11 transmits the sound data to the extraction unit 12.

Note that the sound acquiring unit 11 may transmit, to the extraction unit 12, divided sound data obtained by analyzing the sound data acquired from the detection apparatus 2 to divide the sound data into predetermined segments. The "predetermined segment" may be, for example, a segment obtained by dividing the sound data by a human respiration period (e.g., every series of actions of inhaling and exhaling). The "predetermined segment" may be, for example, a segment obtained by dividing the obtained sound data by any time period (e.g., a segment of every 30 seconds from the start of the detection). Note that the predetermined segment is not limited to these examples. In other words, the sound acquiring unit 11 may transmit, to the extraction unit 12, the sound data divided appropriately in a range used for constructing the learned model 24. That is, the predetermined segment may be, for example, a segment obtained by dividing the sound data every two times a human performs a series of actions of inhaling and exhaling. The sound acquiring unit 11 may select any data from the divided sound data and transmit the selected data to the extraction unit 12, for example. Specifically, the sound acquiring unit 11, in a case of acquiring sound data of a 180-second period, may transmit, to the extraction unit 12, data from the start of the detection until 60 seconds after the start of the detection and data from 120 seconds after the start of the detection until 180 seconds after the start of the detection, for example. That is, the sound data transmitted by the sound acquiring unit 11 to the extraction unit 12 may be selected as appropriate.

Each divided sound data may include identification information on the sound data before being divided and identification information on the sound data after being divided. In the following description, unless otherwise stated, the "sound data" indicates both the sound data before being divided and the sound data after being divided.

The extraction unit 12 can extract a feature of the sound from the sound data based on the definition of the feature defined by definition data 25 in the storage unit 20. The "feature of the sound" may be a parameter when characteristics of a breath sound is extracted by a method independent from the medical classification of the breath sound described above. The "feature of the sound" may be, for example, a parameter extracted based on at least one of a temporal change in a sound, a frequency component included in a sound, or a spectrogram of a sound. The method of extracting the feature of the sound is described below in detail. The extraction unit 12 may associate the plurality of extracted features with the identification information of the sound data to store in the storage unit 20 as feature data 21. A specific configuration of the feature data 21 will be described below.

The classification information acquiring unit 13 can acquire the first data including the classification information from the external apparatus 3. The classification information acquiring unit 13 may store the classification information included in the acquired first data as classification information 22 in the storage unit 20.

The training data creation unit 14 can create training data from the feature data 21 and the classification information 22. The training data creation unit 14 may read at least some pieces of the feature data 21 from the storage unit 20 and further read the classification information 22 having the same identification information as the read feature data 21 from the storage unit 20. Specifically, the training data creation unit 14 may read the feature data 21 and the classification information 22 based on the same sound data.

The training data creation unit 14 can create training data using the read feature data 21 as input data and the classification information 22 as correct answer data. The number of pieces of the training data created by the training data creation unit 14, that is, the scale of a data set for machine learning, may be appropriately determined in accordance with a structure or the like of the learned model 24 to be constructed. The training data creation unit 14 may store the created training data as training data 23 in the storage unit 20.

The model construction unit 15 can construct the learned model by causing an unlearned learning model to perform machine learning using the training data 23. Note that the unlearned learning model (i.e., a template of the learned model 24) may be retained by the model construction unit 15 or may be stored in the storage unit 20. The specific method of the machine learning in the model construction unit 15 is not particularly limited. The model construction unit 15 may store the constructed learned model as the learned model 24 in the storage unit 20.

Note that the model construction unit 15 need not be necessarily included in the control apparatus 1. For example, the model construction unit 15 may be included in an apparatus different from the control apparatus 1. For example, the model construction unit 15 may be stored in an external server connected to the control apparatus 1. That is, the construction of the learned model may be performed in an apparatus other than the control apparatus 1. In this case, the control apparatus 1 and the other apparatus may be connected by wire or wirelessly, and the information used for constructing the learned model may be transmitted and received as appropriate.

### Storage Unit 20

The storage unit 20 is a storage device storing various types of data used by the control apparatus 1 to operate. The storage unit 20 includes the feature data 21, the classification information 22, the training data 23, the learned model 24, and the definition data 25 which are described above. The structure of the learned model 24 is described below in detail.

The definition data 25 is data defining a type of parameter for the feature extracted by the extraction unit 12 and the extraction method of the parameter. That is, the definition data 25 is data for extracting a plurality of features from the sound data. The definition data 25 may be created by a user of the control system 100 and stored in the storage unit 20 in advance. Note that a method of creating the definition data 25 is not particularly limited.

Note that the definition data 25 need not be necessarily stored in the storage unit 20. For example, the definition data 25 may be stored in an apparatus different from the control apparatus 1. For example, the definition data 25 may be stored in an external server connected to the control apparatus 1. For example, the extraction unit 12 need not be necessarily included in the control apparatus 1. For example, the extraction unit 12 may be stored in an external server connected to the control apparatus 1. That is, the extraction of the feature may be performed in an apparatus other than the control apparatus 1. In this case, the control apparatus 1 and the other apparatus may be connected by wire or wirelessly, and the information used for extracting the feature may be transmitted and received as appropriate.

### Modified Example

In the control system 100, the external apparatus 3 is not an indispensable component. In a case that the control system 100 does not include the external apparatus 3, the control apparatus 1 may have configurations corresponding to various members of the external apparatus 3 described above to achieve functions as the external apparatus 3. The user of the control system 100 such as the specialist may input the classification of the breath sound via the input unit of the control apparatus 1 rather than the external apparatus 3. The classification information acquiring unit 13 may acquire information indicating the classification of the breath sound from the input unit. The same applies to the subsequent processing.

### Method of Extracting Feature

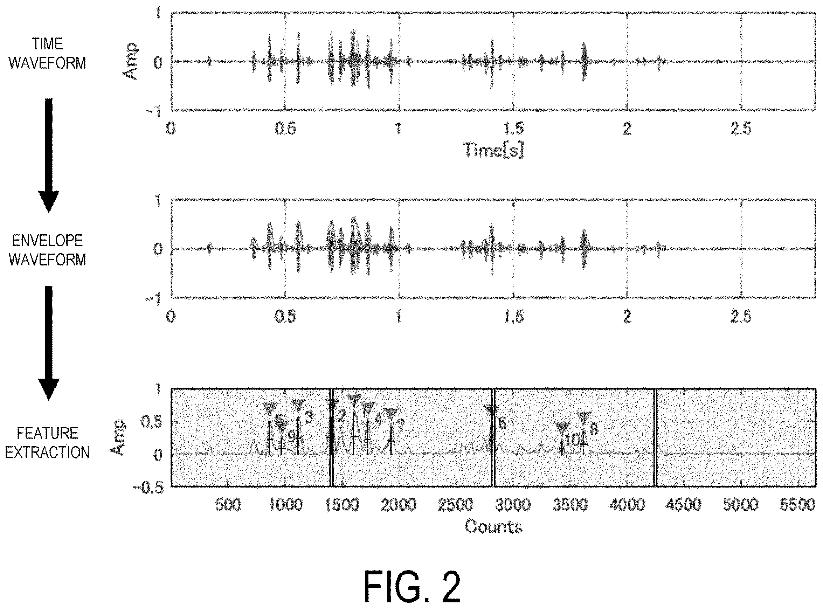

The extraction of the feature in the extraction unit 12 and the structure of the feature data 21 are described in detail using FIGS. 2 to 5. Note that in FIGS. 2 to 4, the sound data is processed in the order illustrated by arrows, and the feature is extracted. Note that while in FIGS. 2 to 4, a time waveform, a frequency waveform, and a spectrogram waveform are illustrated as graphs to facilitate understanding, these waveforms need not necessarily be visualized in the control system 100.

FIG. 2 is a diagram illustrating an extraction process of a feature from a time waveform of sound data. The time waveform indicates a temporal change in an output of a sound. As illustrated in FIG. 2, in the graph of the time waveform, a horizontal axis indicates an output time of the sound, and a vertical axis indicates an output intensity. First, the extraction unit 12 analyzes the sound data to identify a time waveform of the sound data. Next, the extraction unit 12 processes the time waveform into an envelope waveform. Finally, the extraction unit 12 extracts the feature of the sound data from the envelope waveform.

For example, the extraction unit 12 may extract top ten peaks from the envelope waveform to extract values indicated by the peaks on the vertical axis of the graph (hereinafter, also referred to as a peak value) as features. In addition, for example, the extraction unit 12 may extract at least one of time positions of the top ten peaks, a dispersion of the peak values, or an average of the peak values, as features. For example, the extraction unit 12 may identify an envelope width of the top ten peaks and an energy concentration for each of time positions of the top ten peaks to extract these as features. Note that the energy concentration referred to here indicates an area ratio of the waveform in each section obtained by dividing the entire time of the envelope waveform into a predetermined number of sections.

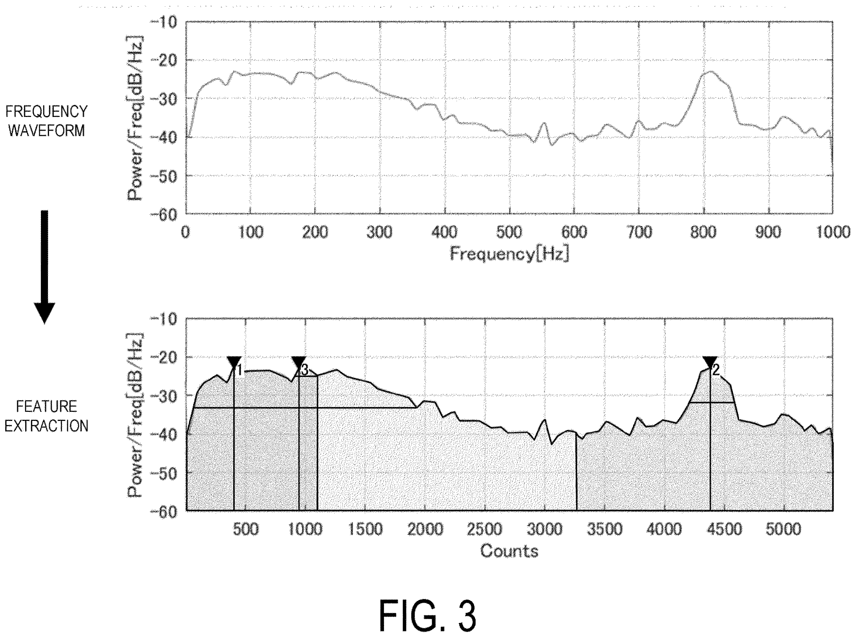

FIG. 3 is a diagram illustrating an extraction process of a feature from a frequency waveform of sound data. The frequency waveform indicates a distribution of frequency components included in certain sound data. As illustrated in FIG. 3, in the graph of the frequency waveform, a horizontal axis indicates a frequency, and a vertical axis indicates an intensity. First, the extraction unit 12 analyzes the sound data to identify a frequency waveform of the sound data. Next, the extraction unit 12 extracts the feature of sound data from the frequency waveform. The frequency waveform can be determined by Fourier transform of the temporal change in an output of a sound.

For example, the extraction unit 12 may extract the top three peaks from the frequency waveform to extract frequency positions of the peaks as features. For example, the extraction unit 12 may identify bandwidths of the top three peaks and an energy concentration for each frequency band to extract these as features. Note that the energy concentration referred to here indicates an area ratio of the waveform in each section obtained by dividing the entire frequency of the frequency waveform into a predetermined number of sections.

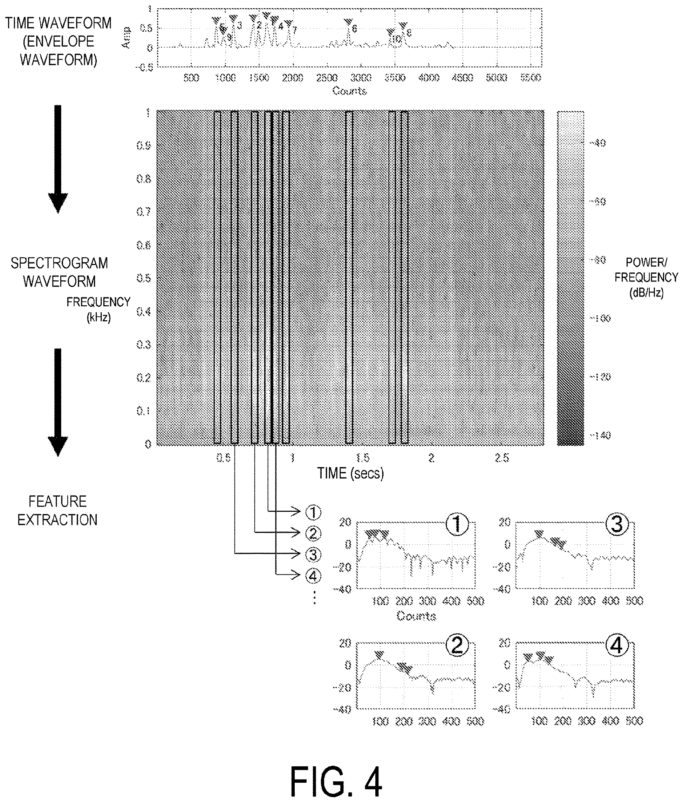

FIG. 4 is a diagram illustrating an extraction process of a feature from a spectrogram waveform of sound data. The spectrogram waveform indicates a temporal change in a frequency component included in certain sound data. As illustrated in FIG. 4, in the graph of the spectrogram waveform, a horizontal axis indicates a time, a vertical axis indicates a frequency, and shades indicate intensities. First, the extraction unit 12 analyzes the sound data to identify a time waveform of the sound data. Next, the extraction unit 12 identifies a spectrogram waveform from the time waveform. Finally, the extraction unit 12 extracts the feature of the sound data from the spectrogram waveform. The spectrogram waveform can be determined by Fourier transform of the time waveform for each predetermined time to calculate the frequency waveform for each time and connect them together.

For example, as illustrated in FIG. 4, the extraction unit 12 may extract the top three peaks from each of the time waveform portions of the spectrogram waveform. The extraction unit 12 may identify the top three frequency peak values at each of the peak time positions, frequency positions, dispersion and average of the positions, a bandwidth, and an energy concentration for each frequency band to extract these as features.

The control apparatus 1 may display the graphs of the time waveform, the frequency waveform, and the spectrogram waveform on the display unit of the control apparatus 1 or a display apparatus connected to the control apparatus 1. In this case, the user visually recognizes the displayed graphs to easily determine a data range used for construction of the learned model 24. That is, the control system 100 can improve convenience.

As illustrated in FIG. 2 to FIG. 4, the definition data 25 is defined to extract the feature based on at least one of the temporal change, frequency component, or spectrogram of the sound data, allowing various features to be extracted from the sound data. Therefore, it is possible to construct the learned model 24 capable of determining the classification of the sound more accurately.

### Data Structure of Feature Data

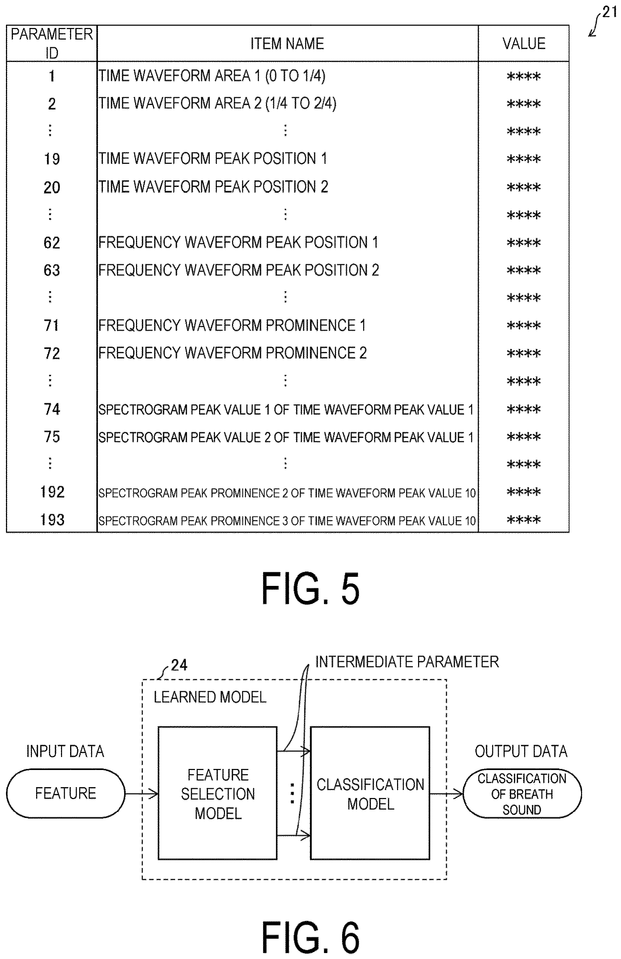

FIG. 5 is a diagram illustrating an example of a data structure of the feature data 21. One row in FIG. 5, that is, one record, indicates an identification number (ID), item name, and value of one feature. Stored in a field of the "item name" is information, defined by the definition data 25, indicating a property, a calculation method, or the like for each feature. Note that for convenience in the example in FIG. 5, the column of "item name" is provided to illustrate the description of the property for each feature, but the column of "item name" is not indispensable in the feature data 21. That is, the feature data 21 is the data in which the identification number capable of uniquely identifying each feature is associated with the value of the feature.

### Structure and Operation Overview of Learned Model 24

FIG. 6 is a schematic view illustrating an overview of input and output data of the learned model 24 and a configuration of the learned model 24. Note that the configuration of the learned model 24 illustrated in FIG. 6 is merely an example, and the configuration of the learned model 24 is not limited thereto.

As illustrated in the figure, the learned model 24 is configured to use the feature of sound data as input data and finally the classification of the sound as output data. The learned model 24 may include, for example, a feature selection model that weights, joins, or sifts through various parameters for the input feature to reduce the total number of parameters.

The feature selection model may be configured with a neural network (NN) or may be configured as an aggregate of one or more model expressions such as polynomials, for example. The feature selection model may perform (1) to (3) below, for example.
(1) Multiplying each of the input features by a weighting coefficient.
(2) Selecting two or more parameters from the multiplied features.
(3) Calculating a sum, a difference, a product, or a quotient of the selected parameters, and combinations thereof.

By doing so, an intermediate parameter obtained by weighting and joining the two or more parameters can be created. The feature selection model may set the weight for one or more features to 0, or sift through one or more features among the input features to reduce the total number of parameters for the features, for example. For example, principal component analysis or independent component analysis may be used to sift through the features. In this way, a plurality of intermediate parameters obtained by performing the weighting, joining, or sifting through in the feature selection model may be input into the next classification model. In this way, a wide variety of features are weighted, joined, or sifted through so that the classification of sound can be determined more accurately in subsequent classification models.

The classification model is a classifier implemented by, for example, a support vector machine (SVM). The classification model identifies and outputs a classification of a sound indicated by the input intermediate parameters. Note that the classification model may also be an NN. In a case that both the feature selection model and the classification model are the NN, these two models may be one NN.

### Flow of Model Constructing Processing

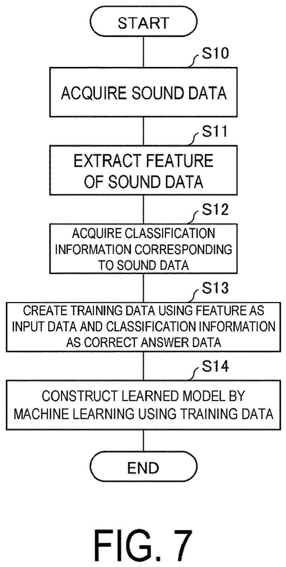

FIG. 7 is a flowchart illustrating a flow of processing (model constructing processing) in which the control apparatus 1 constructs the learned model 24.

First, the control apparatus 1 acquires various pieces of data which are materials of the training data 23. Specifically, the sound acquiring unit 11 acquires sound data of a breath sound from the detection apparatus 2 (S10). The sound acquiring unit 11 outputs the sound data to the extraction unit 12. The extraction unit 12 extracts a feature of the sound data from the input sound data (S11). Here, the extraction unit 12 extracts at least two or more features. The extraction unit 12 stores the various extracted features as the feature data 21 in the storage unit 20.

The classification information acquiring unit 13 acquires, in no particular order from, or in parallel with the processing operations in S10 and S11, the classification information corresponding to the sound data acquired by the sound acquiring unit 11 in S10 from the external apparatus 3 (SI2). The classification information acquiring unit 13 stores the acquired classification information as the classification information 22 in the storage unit 20.

Next, the control apparatus 1 creates the training data 23. Specifically, the training data creation unit 14 reads the feature data 21 and the classification information 22 to create training data that uses the feature as the input data and the classification information as the correct answer data (S13). The training data creation unit 14 stores the created training data as the training data 23 in the storage unit 20. Note that the processing in S13 may be performed at a timing independent from S10 to S12.

Finally, the control apparatus 1 performs the machine learning using the training data 23. Specifically, the model construction unit 15 reads the training data 23 to cause an unlearned learning model to perform machine learning using the training data 23. By doing so, the model construction unit 15 constructs the learned model 24 (S14). The model construction unit 15 stores the constructed learned model 24 in the storage unit 20.

According to the above processing, the control apparatus 1 can construct the learned model 24 where machine learning on the correlation between the feature of the sound data and the medical classification of sound of the sound data is performed. That is, the control apparatus 1 can construct the learned model 24 capable of accurately determining, based on a sound emitted by an inspection target, a classification of the sound.

Conventionally, in a case, for example, that a physician medically classifies a breath sound by way of auscultating, the breath sound has been classified based on the experience of the physician. For example, if a duration of the sound is 200 milliseconds or more, the physician has experientially judged that the breath sound is a continuous rale. For example, the physician has experientially judged that a breath sound, among the continuous rales, mainly including many sounds having a frequency of 200 Hz or less is a low-pitched continuous rale that is referred to as a sonorous rhonchus. For example, the physician has experientially judged that a breath sound, among the continuous rales, mainly containing many sounds having a frequency of 400 Hz or less is a high-pitched continuous rale that is referred to as a wheeze. However, this classification method cannot always correctly classify all breath sounds. For example, a plurality of sounds due to a plurality of disease conditions may be mixed in the breath sound. In this case, the breath sound includes sound of a plurality of frequencies, and thus, the physician may mistake the judgment. In a case that whether a symptom is serious or mild is judged based on the experience of a specialist such as a physician, the judgment on whether the symptom is serious or mild may vary depending on the specialist.

In contrast, the feature extracted in the extraction unit 12 is a feature defined independently from the medical classification of the breath sound included in the classification information. The feature is defined by the definition data 25, which allows the number of features to be freely increased and decreased by the user. Thus, for example, defining more various and larger number of features than the number of medical classifications allows the learned model 24 to be constructed based on such a number of features. Therefore, the learned model 24 can more accurately determine the classification of the breath sound than a physician simply classifying the breath sound. This is because the learned model 24 also learns by machine learning on the relationship between the characteristics of the breath sound, which cannot be taken into account by the known method, and the classification of the breath sound such as a noise in which characteristics of a plurality of classifications are mixed or a noise that does not match the known classification but is actually caused by a disease, for example.

### Second Embodiment

Other embodiments of the present invention will be described below. Note that, for convenience of description, members having the same functions as those described in the above-described embodiment are denoted by the same reference numerals, and descriptions thereof will not be repeated. The same applies to the following embodiments.

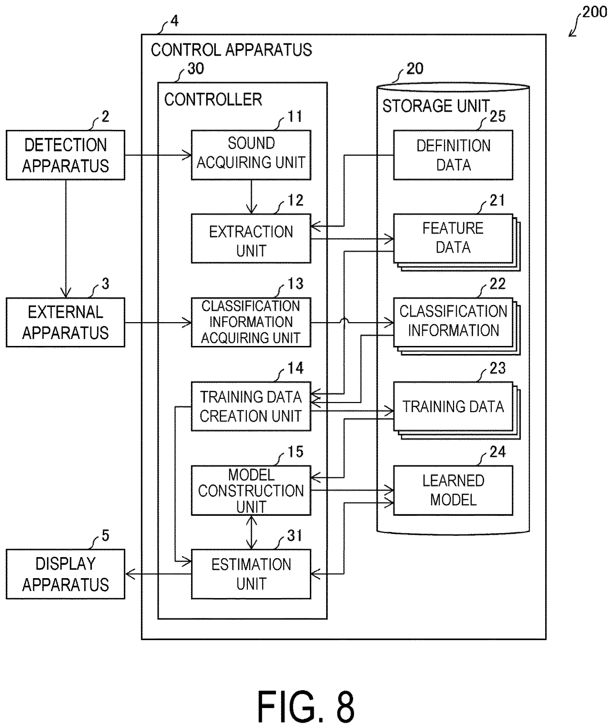

The control apparatus of the control system according to the present invention may include an evaluation unit that evaluates a result of the machine learning of the learned model and provides feedback to the model construction unit. The model construction unit may modify the configuration of the learned model by causing the learned model to perform relearning based on the feedback from the evaluation unit. Hereinafter, a control system 200 according to the present embodiment will be described using FIG. 8.

FIG. 8 is a block diagram illustrating a configuration of a main part of the control system 200 according to the present embodiment. The control system 200 differs from the control system 100 according to the first embodiment in that the control system 200 includes a control apparatus 4 instead of the control apparatus 1 and includes a display apparatus 5. Note that the display apparatus 5 is not an indispensable component in the control system 200.

The control apparatus 4 includes a controller 30 and a storage unit 20. The controller 30 includes the similar functions as the control apparatus 1 according to the first embodiment except that the controller 30 includes an evaluation unit 31. The display apparatus 5 is an apparatus displaying an evaluation result of the evaluation unit 31. A specific configuration of the display apparatus 5 is not particularly limited.

The evaluation unit 31 may acquire at least some pieces of data of the training data 23 from the storage unit 20 or the training data creation unit 14. The evaluation unit 31 may input the input data (i.e., the feature of the sound) of the acquired training data 23 into the learned model 24. The estimation result output when certain input data is input to the learned model 24 may be compared to correct answer data (i.e., classification of the sound) corresponding to the input data.

The evaluation unit 31 can repeat this comparison as many times as the number of pieces of the training data 23. Then, the evaluation unit 31, after completing the comparison of all pieces of acquired training data 23, may calculate a comparison result.

The calculation method of the comparison result is not particularly limited. For example, the evaluation unit 31 may calculate, as the result of the comparison, a match ratio between the estimation result and the correct answer data (i.e., an accuracy of the learned model 24). For example, the evaluation unit 31 may calculate, out of the pieces of training data 23 whose correct answer data is "normal breath sound", a percentage of pieces of training data in which the classification of sound is classified into other than "normal breath sound" (i.e., estimated to be abnormal by mistake) by the learned model 24. For example, the evaluation unit 31 may calculate, out of the pieces of training data 23 whose correct answer data is other than "normal breath sound", a percentage of pieces of training data in which the classification of sound is classified into "normal breath sound" (i.e., estimated to be not abnormal by mistake) by the learned model 24.

The evaluation unit 31 may output (i.e., feedback) the comparison result, that is, the evaluation of the machine learning, to the model construction unit 15. The model construction unit 15 may cause the learned model 24 to perform relearning based on the comparison result. Although the method of relearning is not particularly limited, for example, the model construction unit 15 may read, from the storage unit 20, the training data 23, which is similar to the training data 23 presumed to be erroneously answered by the learned model 24 based on the comparison result described above, and may use the read similar training data 23 as a data set for relearning.

Note that in a case that the comparison result described above is good (the case that the comparison result is good refers to, for example, a case that the accuracy of the learned model 24 is equal to or more than a predetermined value), the model construction unit 15 may not perform relearning. In other words, the model construction unit 15 may perform relearning in a case that the evaluation by the evaluation unit 31 does not meet a predetermined condition and may not perform relearning in a case that the evaluation by the evaluation unit 31 meets the predetermined condition.

According to the configuration described above, the learned model 24 constructed once can be evaluated to modify the configuration of the learned model 24 depending on the evaluation. Thus, the learned model 24 can be tuned to a learned model with a higher estimation accuracy.

### Third Embodiment

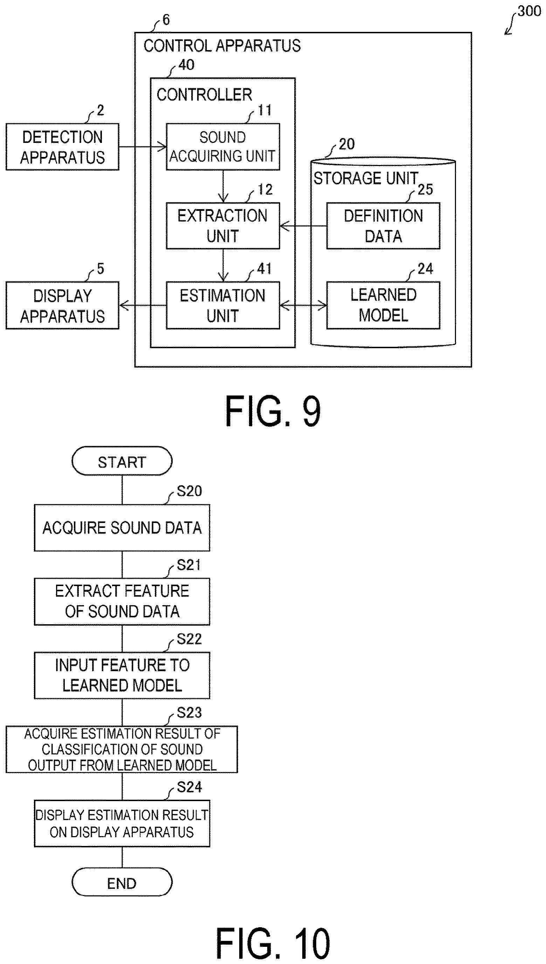

FIG. 9 is a block diagram illustrating a configuration of a main part of a control system 300 according to the present embodiment. The control system 300 includes the detection apparatus 2, the display apparatus 5, and a control apparatus 6. Note that the display apparatus 5 is not an indispensable component in the control system 300.

The control apparatus 6 includes a controller 40 and a storage unit 50. The controller 40 comprehensively controls the control apparatus 6. The controller 40 includes the sound acquiring unit 11, the extraction unit 12, and the estimation unit 41. The storage unit 50 may store the definition data 25 and the learned model 24.

In the present embodiment, the detection apparatus 2 can transmit sound data recording a breath sound of a human as an inspection target to the sound acquiring unit 11. The sound acquiring unit 11 may acquire the sound data and segment the data as necessary to output the segmented data to the extraction unit 12. The extraction unit 12 may extract the feature of the input sound data based on the definition of the definition data 25 to output the feature to the estimation unit 41.

The estimation unit 41 may use the learned model 24 to estimate the classification of the breath sound from the feature of the sound data. The estimation unit 41 may input, into the learned model 24, the feature input from the extraction unit 12 and acquire the estimation result of the classification of the sound output from the learned model 24. The estimation unit 41 may display the estimation result on the display apparatus 5. In the present embodiment, the display apparatus 5 can display the estimation result of the estimation unit 41.

Note that the estimation result itself of the learned model 24, the method of processing the estimation result in the estimation unit 41 using the learned model 24, and the like are not particularly limited. For example, the learned model 24 may be configured to output only one name of a classification of a sound corresponding to the feature or may be configured to output a plurality of names of classifications of a sound corresponding to the feature, depending on the input feature. In a case that a plurality of names of the classifications of the sound are output, the learned model 24 may output a value indicating a degree of matching to each of the classifications of the sound (i.e., a likelihood of the classification of the sound) as the estimation result. In a case that the estimation unit 41 can acquire a plurality of degrees of matching to the plurality of classifications of the sound from the learned model 24, the estimation unit 41 may process the plurality of degrees of matching into a graphic such as a radar chart to be displayed on the display apparatus 5.

### Estimation Processing

FIG. 10 is a diagram illustrating a flow of estimation processing in which the control apparatus 6 estimates a classification of a breath sound. The sound acquiring unit 11 acquires, from the detection apparatus 2, sound data of a breath sound to be inspected (S20). The sound acquiring unit 11 outputs the sound data to the extraction unit 12. The extraction unit 12 extracts a feature of the sound data from the input sound data (S21). The extraction unit 12 outputs the extracted various features to the estimation unit 41.

The estimation unit 41 inputs the feature into the learned model 24 in the storage unit 50 (S22). The learned model 24 outputs the classification of the sound estimated from the feature to the estimation unit 41. The estimation unit 41 acquires the estimation result of the classification of the sound output from the learned model 24 (S23). The estimation unit 41 displays the estimation result on the display apparatus 5 (S24). Note that the processing in S24 is not indispensable. The estimation unit 41 may store the estimation result in the storage unit 50.

According to the processing described above, the control apparatus 1 can use the learned model 24 to estimate, from sound data of a breath sound as an inspection target, a classification of the breath sound. Here, the feature extracted in the extraction unit 12 is a feature, which is included in the classification information, defined independently from the medical classification of the breath sound, and the number of features is more than the number of the medical classifications. The estimation processing is performed based on a large number of features, so that the control apparatus 6 can accurately determine the classification of the sound based on the sound emitted by the inspection target, as compared to simply classifying the breath sound by the physician. The control apparatus 6 can estimate the classification of the breath sound with taking into account the characteristics of the breath sound which cannot be taken into account by the known method, such as a noise in which characteristics of a plurality of classifications are mixed or a noise that does not match the known classification but is actually caused by a disease, for example.

Note that the estimation unit 41 need not be necessarily included in the control apparatus 6. For example, the estimation unit 41 may be included in another apparatus connected to the control apparatus 1. For example, the estimation unit 41 may be included in an external server. That is, the estimation of the classification of the breath sound may be performed by an apparatus other than the control apparatus 6. In this case, the control apparatus 6 and the other apparatus may be connected by wire or wirelessly, and the information used for estimating the classification of the breath sound may be transmitted and received as appropriate.

### Fourth Embodiment

The control system according to the present invention may perform both the model constructing processing and the estimation processing. That is, the control system 100 (or the control system 200) and the control system 300 may be integrally configured. Hereinafter, in the present embodiment, an example in which the control system 100 and the control system 300 are integrally configured is described.

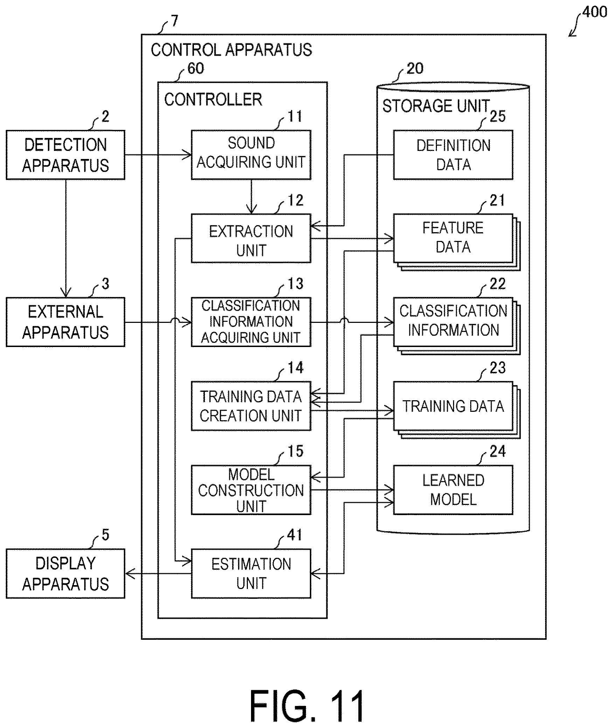

FIG. 11 is a block diagram illustrating a configuration of a main part of a control system 400 according to the present embodiment. The control system 400 includes the detection apparatus 2, the external apparatus 3, the display apparatus 5, and a control apparatus 7. Note that the external apparatus 3 and the display apparatus 5 are not indispensable components also in the control system 400.

The control apparatus 7 includes a controller 60 and the storage unit 20. The controller 60 includes the configuration of the controller 10 according to the control system 100 and the configuration of the controller 40 according to the third embodiment. The control apparatus 7 may perform the model constructing processing described in the first embodiment at any timing to construct the learned model 24. The control apparatus 7 may store the constructed learned model 24 in the storage unit 20. The control apparatus 7 may perform the estimation processing described in the third embodiment at any timing after constructing the learned model 24 to extract the feature from the sound data. Then, the control apparatus 7 may use the learned model 24 to estimate, from the extracted feature, the classification of the sound.

Note that in the case that the control system 200 and the control system 300 are integrally configured, the basic processing flow is the same. In this case, the control system 400 includes the evaluation unit 31 described in the second embodiment in addition to the configuration illustrated in FIG. 11.

### Modified Example 1

The estimation unit 41 according to the third embodiment or fourth embodiment may estimate the degree of matching to the classification of the sound from a plurality of features of the sound. For example, the estimation unit 41 may estimate the name of the classification of the sound and a level of the degree of matching to the classification. In this case, the learned model 24 is configured to output the name of one or more classifications of the sound and values of the degrees of matching to the classifications as the output data. This allows the estimation unit 41 to more precisely estimate the classification of the sound.

### Modified Example 2

The first data according to each embodiment may include information indicating a state of a subject emitting the sound. The information is hereinafter referred to as state information. The learned model 24 according to each embodiment may be a learned model 24 where machine learning on a correlation between at least one of a plurality of features or a classification of a sound, and a state of a subject emitting the sound is performed.

In this case, in the first embodiment or second embodiment, the classification information and the state information may be input to the external apparatus 3 by a specialist. The external apparatus 3 may transmit the first data including the classification information and the state information to the control apparatus 1 or 4. The classification information acquiring unit 13 in the control apparatus 1 or 4 may associate, when acquiring the first data, each of the classification information and the state information included in the first data with the identification information of the sound data to store in the storage unit 20.

Then, the training data creation unit 14 may create training data using the feature data 21, the classification information 22, and the state information. The model construction unit 15 may cause the learned model 24 to perform machine learning based on the training data.

This constructs the learned model 24 where machine learning on a correlation between at least one of the plurality of features of the sound or the classification of the sound, and the state of the subject emitting the sound is performed.

The estimation unit 41 according to the third embodiment or fourth embodiment may use the learned model 24 according to the modified example to estimate a state of the inspection target from the plurality of features of the sound data. The estimation method may be determined in accordance with a learning aspect of the correlation in the learned model 24. For example, assume that the learned model 24 is a learned model where machine learning on a correlation between a plurality of features of a sound and a classification of the sound and on a correlation between the classification and a state of a subject emitting the sound. In this case, the learned model 24 may first estimate a classification of a sound from a plurality of input features. Then, the learned model 24 may further estimate the state of the subject emitting the sound from the estimated classification of the sound. On the other hand, assume that the learned model 24 is a learned model where machine learning on a correlation between a plurality of features of a sound and a classification of the sound and on a correlation between the plurality of features of the sound and a state of a subject emitting the sound. In this case, the learned model 24 may estimate both a classification of a sound and a state of a subject emitting the sound from a plurality of input features. In addition, assume that the learned model 24 is a learned model where machine learning on a correlation between three types of information, a plurality of features of a sound, a classification of the sound, and a state of a subject emitting the sound. In this case, the learned model 24 may estimate at least one of a classification of a sound or a state of a subject emitting the sound from a plurality of input features.

The construction and use of the learned model 24 as described in the modified example make it possible to estimate, from sound data of a breath sound as an inspection target, a state of a subject emitting the sound.

Note that the state information may be information indicating at least one of a symptom or a disease name corresponding to the medical classification. In this case, from the sound data of the breath sound, at least one of a symptom or a disease name of the subject emitting the breath sound can be estimated.

### Implementation Example by Software

The control blocks of the control apparatuses 1, 4, 6, and 7 may be implemented by a logic circuit (hardware) formed in an integrated circuit (IC chip) or the like or may be implemented by software.

In the latter case, the control apparatuses 1, 4, 6, and 7 include a computer that executes instructions of a program that is software to implement each function. The computer includes, for example, one or more processors and a computer-readable recording medium that stores the above program. Then, in the computer, the processor reads the above program from the recording medium and executes the read program to achieve the object of the present invention. As the processor, a central processing unit (CPU) can be used, for example. As the recording medium, a "non-transitory tangible medium" such as, for example, a read only memory (ROM), a tape, a disk, a card, a semiconductor memory, a programmable logic circuit, and the like can be used. Additionally, a random access memory (RAM) for loading the above program may be further provided. The above program may be supplied to the computer via any transmission medium (communication network, broadcast wave, and the like) capable of transmitting the program. Note that one aspect of the present invention may be implemented in the form of data signals embedded in a carrier wave in which the above program is embodied by electronic transmission.

The invention according to the present disclosure is not limited to the embodiments described above. That is, various changes can be made within the scope of the claims. Furthermore, embodiments that are made by appropriately combining technical means disclosed according to the different embodiments are also included in the technical scope of the invention according to the present disclosure. It should be noted that those skilled in the art can easily make various variations or modifications based on the present disclosure. Accordingly, it should be noted that these variations or modifications are included within the scope of the present disclosure.

### Reference Signs List

1, 4, 6, 7 Control apparatus
2 Detection apparatus
3 External apparatus
5 Display apparatus
10, 30, 40, 60 Controller
11 Sound acquiring unit
12 Extraction unit
13 Classification information acquiring unit
14 Training data creation unit
15 Model construction unit
20, 50 Storage unit
21 Feature data
22 Classification information
23 Training data
24 Learned model
25 Definition data
31 Evaluation unit
41 Estimation unit
100, 200, 300, 400 Control system

## Claims

1. A control apparatus comprising:
a first data acquiring unit configured to acquire first data comprising information indicating a sound classification of a sound;
a second data acquiring unit configured to acquire second data comprising sound information of the sound;
a storage unit configured to store definition data, for extracting a plurality of features from the second data, of the plurality of features;
an extraction unit configured to extract the plurality of features of the second data based on the definition data; and
a model construction unit configured to construct a learned model where machine learning, based on the plurality of features of the second data and the first data, on a correlation between the plurality of features and the sound classification is performed.

2. The control apparatus according to claim 1, wherein
the learned model is a model capable of estimating a correlation between a plurality of parameters and the sound classification, the plurality of parameters being obtained by weighting, joining, or sifting through the plurality of features of the second data.

3. The control apparatus according to claim 1 or 2, further comprising
an evaluation unit configured to evaluate a result of the machine learning performed on the learned model to provide feedback to the model construction unit, wherein
the model construction unit is configured to cause the learned model to perform relearning based on the feedback from the evaluation unit.

4. The control apparatus according to any one of claims 1 to 3, wherein
in the definition data, the plurality of features are defined based on at least one of a temporal change in the second data, a frequency component of the second data, or a spectrogram of the second data.

5. The control apparatus according to any one of claims 1 to 4, wherein
the sound classification comprises a medical classification of a breath sound.

6. The control apparatus according to any one of claims 1 to 5, wherein
the first data comprises information indicating a state of a subject emitting the sound, and
the model construction unit is configured to construct a learned model where machine learning, based on the plurality of features and the first data, on a correlation between at least one of the plurality of features or the sound classification, and the state of the subject emitting the sound is further performed.

7. A control apparatus comprising:
a second data acquiring unit configured to acquire second data comprising information of a sound of an inspection target;
a storage unit configured to store definition data, for extracting a plurality of features from the second data, of the plurality of features;
an extraction unit configured to extract the plurality of features of the second data based on the definition data; and
an estimation unit configured to estimate a classification of the sound of the inspection target from the plurality of features of the second data by using a learned model where machine learning on a correlation between the plurality of features and the classification of the sound is performed.

8. The control apparatus according to claim 7, wherein
the estimation unit is configured to estimate a degree of matching to the classification of the sound from the plurality of features.

9. The control apparatus according to claim 7 or 8, wherein
the learned model is a learned model where machine learning on a correlation between at least one of the plurality of features or the classification of the sound, and a state of a subject emitting the sound is further performed, and
the estimation unit is configured to estimate a state of the inspection target from the plurality of features of the second data by using the learned model.

10. The control apparatus according to claim 9, wherein
the classification of the sound is a medical classification of a breath sound, and
information indicating the state of the subject is information indicating at least one of a symptom or a disease name corresponding to the medical classification.

11. A control system comprising:
the control apparatus according to any one of claims 1 to 10; and
a detection apparatus configured to transmit the second data detected to the control apparatus.

12. A control method comprising the steps of:
acquiring first data comprising information indicating a sound classification of a sound;
acquiring second data comprising sound information of the sound;
storing definition data, for extracting a plurality of features from the second data, of the plurality of features;
extracting the plurality of features of the second data based on the definition data; and
constructing a learned model where machine learning, based on the plurality of features of the second data and the first data, on a correlation between the plurality of features and the sound classification is performed.

13. The control method according to claim 12, wherein
the learned model is a model capable of estimating a correlation between a plurality of parameters and the sound classification, the plurality of parameters being obtained by weighting, joining, or sifting through the plurality of features of the second data.

14. The control method according to claim 12 or 13, further comprising the steps of:
evaluating a result of the machine learning performed on the learned model to provide feedback to the constructing step; and
causing the learned model to perform relearning based on the feedback.

15. The control method according to any one of claims 12 to 14, wherein
the first data comprises information indicating a state of a subject emitting the sound, and
in the constructing step, a learned model where machine learning on a correlation between at least one of the plurality of features or the sound classification, and the state of the subject emitting the sound is further performed is constructed based on the plurality of features and the first data.

16. A control method comprising the steps of:
acquiring second data comprising information of a sound of an inspection target;
storing definition data, for extracting a plurality of features from the second data, of the plurality of features;
extracting the plurality of features of the second data based on the definition data; and
estimating a classification of the sound of the inspection target from the plurality of features of the second data by using a learned model where machine learning on a correlation between the plurality of features and the classification of the sound is performed.

17. The control method according to claim 16, wherein
the learned model is a learned model where machine learning on a correlation between at least one of the plurality of features or the classification of the sound, and a state of a subject emitting the sound is further performed, and
in the estimating step, a state of the inspection target is estimated from the plurality of features of the second data by using the learned model.
